# EUROPEAN PATENT SPECIFICATION

(11) **EP 3 037 098 B1**
(45) Date of publication and mention of the grant of the patent: **26.03.2025**
(21) Application number: 15202007.9
(22) Date of filing: 22.12.2015
(51) Int. Cl.: A61K 31/736, A61K 33/06, A61K 33/12, A61P 3/10, A61K 9/16

(54) **COMPOSITION FOR THE TREATMENT AND PREVENTION OF A DISORDER CORRELATED WITH HAEMATIC CHOLESTEROL AND /OR HEMATIC GLYCEMIA**
ZUSAMMENSETZUNG ZUR BEHANDLUNG UND PRÄVENTION VON ERKRANKUNGEN IN VERBINDUNG MIT HÄMATISCHEM CHOLESTERIN UND HÄMATISCHER GLYKÄMIE
COMPOSITION POUR LE TRAITEMENT ET LA PREVENTION DES MALADIES LIEES AU CHOLESTEROL HEMATIQUE ET A LA GLYCEMIE HEMATIQUEE

(30) Priority: 22.12.2014 IT MI20142205
(43) Date of publication of application: 29.06.2016
(73) Proprietor: Martelli, Mario, 35126 Padova (IT); Bergamaschi, Alessandro, 20123 Milano (IT)
(72) Inventor: Martelli, Mario, 35126 Padova (IT); Bergamaschi, Alessandro, 20123 Milano (IT)
(74) Representative: Palladino, Saverio Massimo

(56) References cited:
- RU-C1- 2 109 516
- RU-C1- 2 144 360
- US-A1- 2004 175 436
- US-A1- 2005 245 433
- J.N. TRAILOVIC ET AL: "In vitro and in vivo protective effects of three mycotoxin adsorbents against ochratoxin A in broiler chickens", BRITISH POULTRY SCIENCE, vol. 54, no. 4, 1 August 2013 (2013-08-01), pages 515 - 523, XP055178790, ISSN: 0007-1668, DOI: 10.1080/00071668.2013.798627
- EFSA PANEL ON DIETETIC PRODUCTS ET AL: "Scientific Opinion on the substantiation of health claims related to glucomannan and maintenance of normal blood cholesterol concentrations (ID 836, 1560) pursuant to Article 13(1) of Regulation (EC) No 1924/2006", EFSA JOURNAL, 1 October 2009 (2009-10-01), pages 1 - 14, XP055840995, Retrieved from the Internet <URL:https://efsa.onlinelibrary.wiley.com/doi/pdfdirect/10.2903/j.efsa.2009.1258> [retrieved on 20210914], DOI: 10.2903/j.efsa.2009.1258

## Description

### TECHNICAL FIELD

The present invention concerns a composition for preventing or treating disorders correlated with excess haematic cholesterol and glycemia.

The present invention originates in the sector of nutraceutical, dietary and pharmaceutical products.

In particular the present invention concerns a pharmaceutical or dietary composition which can be applied to regulating or reducing the total haematic cholesterol values.

### PRIOR ART

Cholesterol is a lipid molecule which belongs to the class of the sterols, constitutes an essential component of the cell membrane in animal cells and is involved in cell growth and division. Cholesterol furthermore constitutes the precursor of steroid hormones, bile acids and vitamin D.

Cholesterol, being insoluble in water, cannot circulate freely in the haematic plasma but needs to bind with specific proteins called apolipoproteins (APO) forming complex structures. When cholesterol is referred to in medical terms, it is not the molecule in itself that is referred to but rather the molecule bound with the apolipoproteins to form complexes. These complexes are formed of apolipoproteins, cholesterol, triglycerides and phospholipids.

These lipoproteins are divided into 4 main groups according to their density: very low density lipoproteins (VLDL), low density lipoproteins (LDL) intermediate density lipoproteins (IDL) and high density lipoproteins (HDL).

Generally the low density lipoproteins (LDL) carry between 60% and 80% of the serum cholesterol. Having a high affinity with the artery endothelium cells, they release the cholesterol onto the vessel walls, favouring formation of the atheromatous plaque in atherosclerosis; vice versa the high density lipoproteins (HDL) perform the opposite function, removing the cholesterol from the arteries and taking it back to the liver.

The set of haematic values of these 4 lipoprotein fractions constitutes a critical indicator for evaluating an individual's health, diagnosing possible dyslipidemia and evaluating cardiovascular risk.

The presence of total serum cholesterol with values higher than 200 mg/100 ml plasma represents an important index and a threshold value above which the doctor must consider recourse to pharmacological remedies or treatment.

In patients suffering from slight hypercholesterolemia, the first approach is to intervene in terms of diet and physical activity in order to reduce the total haematic cholesterol content and increase the cholesterol associated with the HDL. These measures are designed to reduce the general cardiovascular risk factor.

When these measures do not determine a reduction in the haematic cholesterol values, pharmacological intervention is necessary or the administration of dietary products with hypocholesterolemizing action.

The dietary products with hypocholesterolemizing action currently in use, for example the phytosterols, monacolins and some vegetable extracts, have the advantage of being of vegetable origin or in any case non-synthetic and being substantially without side effects.

However, in numerous cases it has been observed that the administration of these products of natural or in any case non-synthetic origin does not determine a significant reduction in the total haematic cholesterol.

The use of synthetic pharmaceutical products such as gemfibrozil, fenofibrate and statins has, vice versa, the advantage of determining a significant reduction in the total haematic cholesterol values.

However, the administration of synthetic hypocholesterolemizing products is not without side effects, which can be significant. It has been widely documented in literature that the chronic use of statins can determine liver toxicity and is often associated with myopathy and rhabdomyolysis.

Some documents have proposed for the purpose the use of mixtures containing zeolites.

Patent RU 2144360 C1 reports the use of a product obtained from the grinding of tuff, containing zeolites in a non-specified quantity together with other components of the rock as hypocholesterolemic agent.

Patent RU 2109516 reports the use of a mixture of zeolites (in particular heulandite) and wheat bran for removing excess cholesterol.

Patent application US 2004/0175436 A1 describes the use of mixtures of synthetic zeolites and proteins extracted from herbs showing a hypoglycemia effect.

Patent application US 2005/0245433 A1 is directed to the use of cysteamine for the treatment of hypercholesterolemia, the prevention of the onset of diabetes, and the treatment of complications associated to diabetes. This document discloses that cysteamine can be administered to patients in various "host materials", among which zeolite, to provide a solid form of cysteamine.

The paper "In vitro and in vivo protective effects of three mycotoxin adsorbents against ochratoxin A in broiler chickens", J.N. Trailovic et al., British Poultry Science, vol. 54, no. 4, August 2013, pages 515-523, discloses the possible use of zeolites alone, of an esterified glucomannan alone, or of the two compounds in combination with enzymes, for the absorption of mycotoxins.

Finally, EFSA Journal 2009; 7(9):1258, reports a scientific opinion on the effects of glucomannan in the maintenance of normal blood cholesterol concentrations.

The need is still felt in the sector for remedies and preparations of non-synthetic origin suited to preventing and treating metabolic disorders correlated with haematic cholesterol, in particular hypercholesterolemia, hyperlipidemia, metabolic syndrome, heart diseases, cardiovascular diseases and in general dyslipidemia which occur with or accompany high levels of haematic cholesterol.

A general object of the present invention is to provide the use of a product of natural origin to control or reduce the levels of haematic cholesterol and improve the clinical picture connected with hypercholesterolemia.

A further object of the present invention consists in providing a composition which has hypocholesterolemizing activity and is of non-synthetic origin and the administration of which is almost without side effects on the human organism.

A further object of the present invention is a preparation suitable for treating and/or preventing disorders correlated with haematic cholesterol which allows long-term treatment in the absence of toxic effects on the human organism.

### SUMMARY OF THE INVENTION

The present invention is defined in the appended claims.

The present invention originates from having ascertained the importance of ionic exchange in regulation at haematic level, and in control of the absorption, of some specific nutrients such as cholesterol and sugars, the presence of which in the blood in quantities higher than the physiological levels can cause disorders in the human organism.

According to some aspects of the invention, the inventors have surprisingly discovered that by administering a composition comprising a zeolite and glucomannan, to an individual suffering from a disorder correlated with haematic cholesterol, a reduction is obtained in the cholesterol and/or glycemia values and/or a reduction in the quantity of cholesterol/sugars that can be absorbed in the digestive tract of the human organism.

According to a first aspect of the present invention, a composition comprising a zeolite and glucomannan is provided for use in the treatment or prevention of a disorder correlated with haematic cholesterol.

In particular the present invention provides said composition for hypoglycemizing and/or hypocholesterolizing use.

Typically the composition of the invention comprises a physiologically or pharmacologically acceptable carrier.

Within the composition the glucomannan performs a dual action as hypocholesterolemizing agent and as an agent that increases the palatability of the composition.

The inventors have furthermore discovered that by combining zeolite with glucomannan a synergism of hypoglycemizing and/or hypocholesterolizing action is obtained in the individuals treated.

According to some embodiments, the composition of the invention comprises further active substances, for example having hypocholesterolemizing activity, such as psyllium, pectin and mixtures thereof.

Typically in the composition of the invention the active components with synergic hypocholesterolemizing activity are provided in a pharmacologically effective quantity.

The composition of the invention can be applied in the prevention and/or treatment of disorders correlated with haematic cholesterol or as adjuvant of the symptomatology associated with these affections.

According to some embodiments, the composition of the invention is a pharmaceutical composition, a food supplement or a neutraceutical product which can be introduced into the diet of an individual suffering from disorders correlated with haematic cholesterol.

According to some aspects of the invention, a dietary preparation or kit is provided containing zeolite and glucomannan as a combined preparation for simultaneous, separate or subsequent use in the prevention or treatment of disorders correlated with haematic cholesterol or to regulate the total haematic cholesterol value.

### DETAILED DESCRIPTION OF THE INVENTION

According to one aspect of the present invention, the inventors have surprisingly discovered that a composition comprising a zeolite and glucomannan has a treatment or prevention action on a disorder correlated with haematic cholesterol.

Specifically, the inventors have surprisingly discovered that said composition has a hypoglycemizing and/or hypocholesterolemizing action when administered orally to the human organism.

According to a first aspect, the present invention therefore provides a composition comprising a zeolite and glucomannan for use in the treatment or prevention of a disorder correlated with haematic cholesterol.

In the ambit of the invention, the term disorder correlated with haematic cholesterol includes one or more of the following disorders or affections: hyperlipemia, hypercholesterolemia, metabolic syndrome, heart disease, coronary syndrome, cardiovascular disease, myocardial infarction, diabetes, hypertriglyceridemia, hyperglycemia and dyslipidemia which can occur, for example, via a total haematic cholesterol level higher than the physiological value of 200 mg/dl, VLDL and LDL levels higher than the physiological levels.

According to another aspect, the invention concerns a composition for use in the treatment or prevention of a disorder correlated with haematic cholesterol in which said composition comprises zeolites and glucomannan in an effective quantity and a physiologically or pharmacologically acceptable carrier.

The present invention focuses in particular on the use of compositions comprising zeolites and glucomannan in the control and treatment of cholesterolemia since the latter, when it reaches a total value higher than 200 mg/dl, represents an ascertained risk factor for cardiovascular diseases and in particular for cardiac infarction.

The zeolites that can be used in the ambit of the invention comprise crystalline microporous solids with defined structure that contain silicon, aluminium and oxygen. The zeolite can contain water and/or other molecules within its pores.

According to some embodiments, the zeolites of the invention are those with a Si/Al ratio below 2.5, which typically have a high cation exchange capacity; it is also possible to use zeolites with Si/Al ratio above 2.5, in this case in a higher quantity, or polymethylsiloxanes with porous structure, for example Enterosgel^{™}.

In some embodiments of the invention, synthetic zeolite can be used, i.e. obtained by means of chemical process.

According to some embodiments, the zeolite contains minerals having the general formula:

MₓO.Al₂O₃.YSiO₂.ZH₂O, (A)

in which:
M represents sodium, potassium or calcium,
x is 1 or 2,
Y is between 3 and 10 and is preferably 3 or 10,
Z is 6 or 7.

In certain embodiments the zeolites with formula (A) have a Si/Al ratio below 2.5.

According to some embodiments, the zeolite has a mean particle size ranging from 0.001 to 0.5 mm, preferably 0.09 to 0.25 mm; the desired granulometric fractions can be obtained without difficulty by methods known in the field of grinding rocks or granulates with larger size (traditional grinding, counter-rotating grinding wheels, jet-milling, ...) and subsequent sieving, with mechanical sieves, of the powder obtained from the grinding.

According to certain embodiments, suitable zeolites have the following composition: chabazite and phillipsite from 45 to 75%, sanidine from 10 to 30%, biotite from 10 to 20%, analcime from 2 to 15%, all the percentages being expressed by weight.

By way of example a suitable zeolite has the following composition: chabazite and phillipsite 60%, sanidine 18%, biotite 15%, analcime 7% by weight.

In some embodiments, the composition of the invention contains zeolite in a quantity ranging from 0.001 to 10 g, from 0.01 to 5 g and from 0.5 to 3 g.

In certain embodiments the composition of the invention contains zeolites in a quantity ranging from 0.01 to 90% by weight, from 0.1 to 80% by weight or from 1 to 75% by weight.

According to some embodiments, the present invention provides a composition comprising a zeolite and glucomannan to reduce the level of total cholesterol and/or glycemia or to regulate it to values close to the physiological values equal to 200 mg/dl for cholesterol and 100 mg/dl for glycemia, both values verified on an empty stomach for at least three hours by means of conventional blood test.

The inventors observed that the effect of regulation or reduction of the haematic cholesterol values obtained with the administration of zeolites is unexpectedly increased by its combination with glucomannan.

Furthermore it was surprisingly observed that by formulating the zeolites in combination with glucomannan, the palatability of the resulting mixture or composition is increased.

The presence of glucomannan in the composition not only makes the zeolite more palatable but a synergic activity of reduction of the total haematic cholesterol and/or the glycemia value is also obtained. This synergic effect allows the use of a quantity of glucomannan equal to half the minimum suggested by the EFSA (European Safety Authority Journal 2009; 7(9), 1258) to achieve reduction of the total haematic cholesterol.

In some embodiments, the composition of the invention can comprise glucomannan in a quantity ranging from 10 to 8000 mg, from 100 to 1200 mg or from 300 to 800 mg.

In certain embodiments, the composition of the invention can comprise glucomannan, in a quantity ranging from 0.001 to 30% by weight, from 0.01 to 15% by weight or from 0.1 to 10% by weight.

In some embodiments, the composition of the invention can also comprise further substances or active ingredients.

In some embodiments, the composition of the invention can also comprise one or more further hypocholesterolemizing agents of natural or synthetic origin.

Suitable hypocholesterolemizing agents comprise fermented red rice extracts, policosanols, monacolins, Garcinia-based vegetable extracts (Garcinia cambogia), guggul (Commiphora wightii), turmeric, artichoke, dandelion, garlic or psyllium.

In some embodiments, the composition of the invention also comprises one or more vitamins, for example niacin, vitamin A, vitamin C, vitamin PP and, preferred, the group B vitamins.

According to some embodiments, the composition of the invention also comprises one or more micronutrients and/or minerals such as salts of Mg, K, Na, Zn, Fe, Cr, Se, Mn and others.

The term "carrier" as used herein indicates a means, excipient or diluent with which the association of therapeutic or active ingredients is administered.

Any carrier and/or excipient suitable for the preparation form desired for administration to human beings can be used with the compounds described in the present invention.

For the purposes of the present application, the term "physiologically acceptable" indicates edible substances which are approved by the health authorities for use in pharmaceutical, nutritional or food applications.

In the ambit of the present application, by the term combination it is meant that one or more active ingredients are added to or mixed with one or other ingredients. The term combination should therefore not be understood in the sense that the active ingredients combine with one another with the formation of chemical or other types of bonds.

A physiologically acceptable carrier can be a pharmacologically acceptable carrier. The compositions of the present invention comprise any composition produced by administering the association of active ingredients of the present invention and a physiologically or pharmacologically acceptable carrier. Said compositions are suited to food, nutritional, pharmaceutical or dietary use in mammals, in particular in human beings.

According to some embodiments, the composition of the invention is a food for special medical purposes.

The composition of the invention can take a wide variety of preparation forms, according to the desired administration route.

For example, for oral administration, the composition can be in solid form, for example tablet, capsule, powder, granule or formulations for prolonged release of the active ingredients.

The compositions in solid form, in particular in granular or powder form, are preferred to other types of preparations.

The preparations in solid form can comprise one or more carriers, for example starches, sugars, microcrystalline cellulose, and optionally diluents, granulation agents, lubricants, ligands and disintegrating agents.

The tablets, pills, capsules and granulates can also contain a ligand such as tragacanth, acacia, maize starch or gelatin; excipients such as dicalcium phosphate; a disintegrating agent such as maize starch, potato starch, alginic acid; a lubricant such as magnesium stearate; a sweetening agent such as sucrose, lactose or saccharin. If desired, the tablets can be coated by means of traditional techniques.

When the unitary pharmaceutical form is a capsule, it can contain, in addition to the materials of the above-mentioned type, a liquid carrier such as a fatty oil.

In the case of preparations in liquid form for oral administration, as in the case of suspensions, emulsions and solutions, a suitable carrier can be chosen from water, glycols, oils, alcohol and mixtures thereof.

Typically in the case of liquid preparations, zeolite and glucomannan are in the form of a suspension in water.

Flavourings, preservatives, colourings and similar may also be present in the composition.

In some embodiments, the active ingredients contained in the composition of the present invention can be combined or mixed as active ingredients in intimate mixture with a suitable edible carrier and/or an excipient according to the techniques of the pharmaceutical and food industry or traditional nutrition techniques.

The compositions for pharmaceutical, dietary or nutritional use can be adequately presented in a single pharmaceutical form and prepared by means of any one of the methods well known in pharmaceutical or food technology.

In some embodiments the compositions or preparations of the invention can contain at least 0.1% of each ingredient/active substance. The percentage of zeolite in these compositions can obviously vary, for example from approximately 1% to approximately 95% of the weight of the unit. The quantity of active compound in said compositions is such that a prophylactically or therapeutically effective dose will be obtained.

In some embodiments, the composition of the invention further comprises one or more additional components such as additives, fillers, stabilizers, emulsifiers, texturizers, film-forming agents, plasticizers, wetting agents and thickeners.

Various other materials can be present as coatings or to modify the physical form of the pharmaceutical unit. For example, the tablets can be coated with shellac, sugar or both.

To prevent breakdown as it passes through the upper part of the gastrointestinal tract, the composition can be a formulation with enteric coating.

A syrup or elisir can contain, in addition to the active ingredient, sucrose as a sweetening agent, methyl and propylparabens as preservatives, a colouring agent and a flavouring such as cherry or orange flavour.

In some embodiments, in the compositions of the present invention, the active ingredients are normally formulated in dosage units. The dosage unit can contain 0.01 g to 20 g of active ingredients, zeolite and glucomannan, per dosage unit for the daily administration.

In certain embodiments of the composition of the invention the dosage unit is a sachet which can contain 1 to 50 g or preferably 5 to 30 g of composition.

In some embodiments, the formulation will contain quantities of zeolite and glucomannan which will depend on the gravity of the metabolic disorder and the correlated symptomatology, the condition, any further treatment in progress, the individual's health and the response to the association of active ingredients.

According to some embodiments, the composition of the invention is a medicine. According to some embodiments, the composition of the invention is a supplement, a nutritional product, a dietary product or a food for special medical purposes.

The inventors have ascertained that the zeolites present in the composition of the invention are not absorbed by the organism, and therefore do not generate adverse events or interactions with any other products possibly taken by the patients.

The following examples are provided mainly to illustrate the present invention and are not intended to limit the protective field as resulting from the attached claims. In the examples all the percentages are by weight unless indicated otherwise.

### EXAMPLE 1 (COMPARATIVE)

Formulation based on zeolites from the volcanic area of Lake Bolsena (Viterbo-Italy) having the following composition:
- chabazite and phillipsite 60%;
- sanidine 18%;
- biotite 15%;
- analcime 7%.

For the purposes of the present invention, this formulation is used by pouring it into water in a quantity ranging from 1 to 9 g and orally administering the suspension obtained.

### EXAMPLE 2 (COMPARATIVE)

### Clinical-experimental trials.

25 patients with high haematic cholesterol and glycemia levels took part in the trial. A formulation was administered to the patients, which was obtained by suspending 3 g of zeolites of Example 1 in 50-70 ml of water. The preparation was taken on an empty stomach, without requiring any food restriction.

The results obtained after 30 days of treatment are given in Table 1. The table shows the total glycemia and cholesterol values, in mg per dl of plasma, measured before the treatment (T1) and after the 30 days of treatment (T2).

**Table 1**

| Patient | Gender | Age | Glycemia (mg/dl) | | Tot. cholesterol (mg/dl) | |
|---|---|---|---|---|---|---|
| | | | T1 | T2 | T1 | T2 |
| F001 | M | 63 | 97 | 78 | 294 | 227 |
| F002 | F | 52 | 108 | 83 | 182 | 196 |
| F003 | M | 29 | 92 | 79 | 206 | 179 |
| F004 | M | 29 | 101 | 83 | 262 | 223 |
| F005 | M | 38 | 109 | 78 | 183 | 168 |
| F006 | F | 60 | 102 | 85 | 278 | 238 |
| F007 | F | 61 | 102 | 88 | 216 | 168 |
| F008 | M | 34 | 106 | 90 | 192 | 146 |
| F009 | F | 33 | 98 | 85 | 185 | 176 |
| F010 | F | 27 | 90 | 73 | 153 | 143 |
| F011 | M | 49 | 83 | 66 | 253 | 226 |
| F012 | F | 50 | 94 | 85 | 250 | 264 |
| F013 | F | 23 | 89 | 79 | 173 | 134 |
| N001 | M | 60 | 124 | 101 | 330 | 258 |
| N002 | F | 30 | 91 | 73 | 168 | 155 |
| N003 | M | 30 | 99 | 85 | 169 | 169 |
| N004 | F | 39 | 82 | 76 | 209 | 186 |
| N005 | M | 50 | 115 | 101 | 238 | 207 |
| N006 | M | 31 | 89 | 77 | 157 | 162 |
| N007 | M | 26 | 101 | 90 | 173 | 173 |
| N008 | F | 58 | 98 | 80 | 294 | 245 |
| N009 | M | 58 | 146 | 138 | 242 | 214 |
| N010 | M | 30 | 92 | 89 | 196 | 205 |
| N011 | F | 31 | 94 | 90 | 225 | 201 |
| N012 | M | 20 | 100 | 89 | 230 | 201 |

From the data reported in the table, a reduction of approximately 14.5% in the glycemia and approximately 11% in the total cholesterol can be observed.

### EXAMPLE 3

A formulation is prepared in granular form for the treatment of hypercholesterolemia and correlated symptomatology, comprising 2 g of glucomannan and 3 g of a zeolite having the following composition:
- chabazite and phillipsite 55%;
- sanidine 21%;
- biotite 19%;
- analcime 5%.

### EXAMPLE 4

### Clinical-experimental trials.

A month after the end of the trial of Example 2, the patients having reported in that trial a total final cholesterol level above 220 mg/dl were treated with the formulation of Example 3 for 30 days. The preparation was taken on an empty stomach without food restrictions, suspending the powder in approximately 50-70 ml of water. This preparation was judged to be much more palatable than the formulation of Example 1.

The results are given in Table 2, which reports the total glycemia and cholesterol values measured at the beginning of the treatment (T2) and after the 30 days of treatment (T3).

**Table 2**

| Patient | Gender | Age | Glycemia (mg/dl) | | Total cholesterol (mg/dl) | |
|---|---|---|---|---|---|---|
| | | | T2 | T3 | T2 | T3 |
| F001 | M | 63 | 79 | 75 | 227 | 205 |
| F004 | M | 29 | 91 | 78 | 223 | 195 |
| F006 | F | 60 | 98 | 78 | 238 | 210 |
| F011 | M | 49 | 66 | 66 | 226 | 205 |
| F012 | F | 50 | 85 | 75 | 264 | 208 |
| N001 | M | 60 | 101 | 80 | 258 | 210 |
| N008 | F | 58 | 80 | 80 | 245 | 218 |

From the data provided in the table, a reduction of approximately 11.3% in the glycemia and approximately 13.7% in total cholesterol can be observed.

### EXAMPLE 5

### Clinical-experimental trials.

The same patients treated as in Example 4 were treated for a further 30 days with a preparation that comprised 5 g of glucomannan and 3 g of the zeolite of Example 3 suspended in 50-70 ml of water. This preparation was judged to be more palatable than the one used in Example 1. The results in terms of total cholesterol reduction are given in Table 3.

**Table 3**

| Patient | Gender | Age | Total cholesterol (mg/dl) | |
|---|---|---|---|---|
| | | | T3 | T4 |
| F001 | M | 63 | 205 | 190 |
| F004 | M | 29 | 195 | 190 |
| F006 | F | 60 | 210 | 195 |
| F011 | M | 49 | 205 | 200 |
| F012 | F | 50 | 208 | 195 |
| N001 | M | 60 | 210 | 195 |
| N008 | F | 58 | 218 | 202 |

The data reported in the table show a further reduction of almost 6% in the haematic quantity of total cholesterol compared to the results obtained after the treatment of Example 4.

### EXAMPLE 6

All the patients showed a reduction in body weight, in particular those who at the beginning of the trial were in the range 98-120 kg showed a weight in the range 83-98 kg at the end of the trial.

### COMMENT ON THE RESULTS

The results of Comparative Example 2 show that zeolites are effective in reducing glycemia and total haematic cholesterol level.

The results of Examples 3 and 4 show that the association of the zeolites with glucomannan has further improved effects, leading to a further reduction in the glycemia and cholesterol levels compared to the results that can be obtained with the zeolites only.

## Claims

1. Composition for use in the treatment and/or prevention of a disorder correlated with excess haematic cholesterol and/or glycemia, comprising a zeolite and glucomannan.

2. Composition for the use according to claim 1, wherein the disorder correlated with excess haematic cholesterol is selected from hyperlipidemia, hypercholesterolemia metabolic syndrome, heart disease, coronary syndrome, cardiovascular disease, myocardial infarction, diabetes, hypertriglyceridemia, hyperglycemia and dyslipidemia.

3. Composition for the use according to any one of the preceding claims, wherein the zeolite has the formula (A)
MₓO.Al₂O₃.YSiO₂.ZH₂O, (A)
wherein:
M represents sodium, potassium or calcium,
x is 1 or 2,
Y is from 3 to 10 and is preferably 3 or 10,
Z is 6 or 7.

4. Composition for the use according to any one of the preceding claims wherein the Si/Al atomic ratio is below 2.5.

5. Composition for the use according to any one of the preceding claims, wherein the zeolite comprises chabazite and phillipsite from 45 to 75%, sanidine from 10 to 30%, biotite from 10 to 20%, analcime from 2 to 15%, all the percentages being expressed by weight, optionally in mixture with other zeolites.

6. Composition for use according to any one of the preceding claims, comprising a pharmaceutically or physiologically acceptable carrier.

7. Composition for use according to any one of the preceding claims further comprising at least one further active component selected from micronutrients, mineral salts, and vitamins.

8. Composition for use according to any one of the preceding claims, in the form of a food supplement, a nutraceutical, a medicine or a food for special medical purposes.

9. Composition for use according to any one of the preceding claims for use in the treatment of hypercholesterolemia or hyperglycemia or to regulate the haematic values of cholesterol and glycemia within physiological parameters.

## Patentansprüche

1. Zusammensetzung zur Verwendung bei der Behandlung und/oder Prävention einer Erkrankung, die mit einem Überschuss an hämatischem Cholesterin und/oder einer hämatischen Glykämie in Verbindung steht, die Zeolith und Glucomannan umfasst.

2. Zusammensetzung zur Verwendung nach Anspruch 1, wobei die Erkrankung in Verbindung mit einem Überschuss an hämatischem Cholesterin ausgewählt ist aus Hyperlipidämie, Hypercholesterinämie, metabolischem Syndrom, Herzkrankheit, Koronarsyndrom, kardiovaskulärer Krankheit, Myokardinfarkt, Diabetes, Hypertriglyceridämie, Hyperglykämie und Dyslipidämie.

3. Zusammensetzung zur Verwendung nach einem der vorhergehenden Ansprüche, wobei der Zeolith die Formel (A) aufweist
MₓO.Al₂O₃.YSiO₂.ZH₂O, (A)
wobei:
M für Natrium, Kalium oder Kalzium steht,
x 1 oder 2 ist,
Y 3 bis 10 ist und vorzugsweise 3 oder 10 ist,
Z 6 oder 7 ist.

4. Zusammensetzung zur Verwendung nach einem der vorhergehenden Ansprüche, wobei das Si/Al-Atomverhältnis unter 2,5 liegt.

5. Zusammensetzung zur Verwendung nach einem der vorhergehenden Ansprüche, wobei der Zeolith 45 bis 75% Chabasit und Phillipsit, 10 bis 30% Sanidin, 10 bis 20% Biotit, 2 bis 15% Analcim, wobei alle Prozentangaben in Gewicht ausgedrückt sind, gegebenenfalls in Mischung mit anderen Zeolithen, umfasst.

6. Zusammensetzung zur Verwendung nach einem der vorhergehenden Ansprüche, umfassend einen pharmazeutisch oder physiologisch verträglichen Träger.

7. Zusammensetzung zur Verwendung nach einem der vorhergehenden Ansprüche, ferner umfassend mindestens eine weitere Wirkkomponente, ausgewählt aus Mikronährstoffen, Mineralsalzen und Vitaminen.

8. Zusammensetzung zur Verwendung nach einem der vorhergehenden Ansprüche in Form eines Nahrungsergänzungsmittels, eines Nutrazeutikums, eines Medikaments oder eines Nahrungsmittels für spezielle medizinische Zwecke.

9. Zusammensetzung zur Verwendung nach einem der vorhergehenden Ansprüche zur Verwendung bei der Behandlung von Hypercholesterinämie oder Hyperglykämie oder zur Regulierung der hämatologischen Werte von Cholesterin und Glykämie innerhalb physiologischer Parameter.

## Revendications

1. Composition à utiliser dans le traitement et/ou la prévention d'un trouble corrélé à un excès de cholestérol hématique et/ou de glycémie, comprenant une zéolite et du glucomannane.

2. Composition pour l'utilisation selon la revendication 1, dans laquelle le trouble corrélé à un excès de cholestérol hématique est choisi parmi l'hyperlipidémie, l'hypercholestérolémie, le syndrome métabolique, les maladies cardiaques, le syndrome coronarien, les maladies cardiovasculaires, l'infarctus du myocarde, le diabète, l'hypertriglycéridémie, l'hyperglycémie et la dyslipidémie.

3. Composition pour l'utilisation selon l'une quelconque des revendications précédentes, dans laquelle la zéolite a la formule (A)
MₓO.Al₂O₃.YSiO₂.ZH₂O, (A)
dans laquelle :
M représente le sodium, le potassium ou le calcium,
x est 1 ou 2,
Y est de 3 à 10 et est de préférence 3 ou 10,
Z est 6 ou 7.

4. Composition pour l'utilisation selon l'une quelconque des revendications précédentes, dans laquelle le rapport atomique Si/Al est inférieur à 2,5.

5. Composition pour l'utilisation selon l'une quelconque des revendications précédentes, dans laquelle la zéolite comprend de la chabazite et de la phillipsite de 45 à 75 %, de la sanidine de 10 à 30 %, de la biotite de 10 à 20 %, de l'analcime de 2 à 15 %, tous les pourcentages étant exprimés en poids, éventuellement en mélange avec d'autres zéolites.

6. Composition à utiliser selon l'une quelconque des revendications précédentes, comprenant un support pharmaceutiquement ou physiologiquement acceptable.

7. Composition à utiliser selon l'une quelconque des revendications précédentes, comprenant en outre au moins un autre composant actif choisi parmi les micronutriments, les sels minéraux et les vitamines.

8. Composition à utiliser selon l'une quelconque des revendications précédentes, sous la forme d'un complément alimentaire, d'un nutraceutique, d'un médicament ou d'un aliment à usage médical spécial.

9. Composition à utiliser selon l'une quelconque des revendications précédentes pour une utilisation dans le traitement de l'hypercholestérolémie ou de l'hyperglycémie ou pour réguler les valeurs hématiques du cholestérol et de la glycémie selon les paramètres physiologiques.
